# EUROPEAN PATENT SPECIFICATION

(11) **EP 0 913 450 B1**
(45) Date of publication and mention of the grant of the patent: **13.11.2002**
(21) Application number: 97914596.8
(22) Date of filing: 03.04.1997
(51) Int. Cl.: C10G 35/06, C07C 13/10, C07C 5/13, C10G 45/58

(54) **PROCESS FOR PREPARING METHYLCYCLOPENTANE-CONTAINING HYDROCARBON**
VERFAHREN ZUR HERSTELLUNG VON METHYLCYCLOPENTANHALTIGEM KOHLENWASSERSTOFF
PROCEDE POUR PREPARER UN HYDROCARBURE CONTENANT DU METHYLCYCLOPENTANE

(43) Date of publication of application: 06.05.1999
(73) Proprietor: JAPAN ENERGY CORPORATION, Tokyo 105-0001 (JP)
(72) Inventor: YAMADA, Teruaki, Japan Energy Corporation, Toda-shi, Saitama 335 (JP); OGAWA, Yasuhito, Japan Energy Corporation, Toda-shi, Saitama 335 (JP); MATSUZAWA, Kenji, Japan Energy Corporation, Toda-shi, Saitama 335 (JP)
(74) Representative: Bryer, Kenneth Robert
(86) International application number: JP9701155
(87) International publication number: WO98044076

(56) References cited:
- JP-A- 7 216 366
- JP-A- 7 278 569
- JP-A- 47 039 107
- JP-A- 51 109 903
- JP-A- 52 024 151
- JP-A- 53 137 893
- US-A- 2 721 226
- US-A- 3 567 796
- US-A- 3 631 117
- US-A- 3 974 061

## Description

### Technical field

The present invention relates to a production method for a hydrocarbon. In particular, the present invention relates to a production method of a gasoline which, by efficiently converting benzene to a methylcyclopentane, possesses a high octane number, has an excellent acceleration responsiveness and horsepower performance of the engine, and reduces the content of benzene which is a hazardous compound.

### Background Art

There is demand for an automobile which reduces hazardous substances contained in the exhaust gas and which has a good fuel consumption. One method of improving fuel consumption is to increase the compression ratio of the engine and thereby improving the thermal efficiency. But, in an engine with a high compression ratio, knocking is more likely to occur, and a gasoline with a higher octane number becomes necessary. In the past, there existed a high octane gasoline which had lead alkyl added, but due to the problem of lead pollution, lead alkyl is no longer used. Because of this, high octane gasolines which are produced by adjusting the components in the gasoline are now produced and sold. Stated more concretely, in order to increase the octane number of gasoline, the mixing amount of the fraction of heavy aromatic hydrocarbons, which are components with a high octane number, has been increasing.

In recent years, the effect of aromatic hydrocarbons, in particular benzene, on the human body has become a concern, and there is demand for decreasing the amount of aromatic hydrocarbons mixed in gasoline. As a result, gasoline with aliphatic hydrocarbons which are light and have high octane numbers as well as gasoline mixed with oxygen containing organic compounds such as methyl-t-butyl ether or the like are on the market.

Furthermore, even in gasoline in general, there is concern over aromatic hydrocarbon content similar to the concern in high octane gasoline. There is demand for decreasing the amount of aromatic hydrocarbons which is mixed in. As a result, gasoline with increased lightweight components of aliphatic hydrocarbons is being sold.

Of course, the gasoline base material which is used to produce such a gasoline must also have a low benzene concentration. Methods for removing benzene from the gasoline base material have been studied. The separation of benzene from the refined oil fraction, which is used as the gasoline base material, is difficult with standard distillation methods. New facilities for sulfolane extraction and the like become necessary. On a global scale, as benzene restrictions become implemented, benzene removal from the refined oil fraction will become prevalent not only domestically but also in other countries. There is danger that the benzene supply amount will increase excessively on the market. Because of this, there is demand for a method which removes benzene efficiently and which converts benzene to a hydrocarbon other than an aromatic hydrocarbon.

Furthermore, as benzene in gasoline is reduced and as high octane aliphatic hydrocarbons or oxygen-containing compounds are added, the power output of the engine is reduced. It becomes clear that the acceleration responsiveness is reduced. A major reason for this is the reduction in heating value per unit volume. In order to prevent the reduction in heating value, increasing the naphthene hydrocarbon content, which has a per volume heat value similar to aromatic hydrocarbons, has been tested. For example, in Japanese Patent Laid-Open Number 5-302090, a gasoline composition containing over 1% of an alicyclic compound with a ring greater than a cyclohexane ring is disclosed. In Japanese Patent Laid-Open Number 6-136372, a gasoline composition containing 10∼90 g/l of a 1,3 cyclohexanediene and/or 1,4 cyclohexanediene is disclosed. But, when adding cyclohexane, the research octane number of cyclohexane is around 83. When adding a large amount, it is difficult to maintain the octane number of the gasoline.

In contrast, among the same alicyclic compounds, methylcyclopentane has a high research octane number of 91. Compared to the chain hydrocarbons, methylcyclopentane has a greater per volume heat value. It is a promising gasoline base material. Furthermore, it is known that methylcyclopentane can be produced by hydrogenating benzene and isomerizing the resultant cyclohexane.

As a method of producing methylcyclopentane from benzene, in US Patent Number 3,644,196, benzene is separated from the fraction which contains, as its main component, a 6-carbon hydrocarbon which is obtained by catalytic reforming. Using a catalyst such as a Ni catalyst supported on diatomaceous earth carrier or the like, cyclohexane is created from benzene. Further using a catalyst such as a platinum supported alumina catalyst or the like, methylcyclopentane is produced. In the reaction of the first step, the temperature is approximately 200 degrees C and pressure is approximately 35 kgf/cm². In the reaction of the second step, the temperature is approximately 260 degrees C and pressure is approximately 35kgf/cm². Because it is a 2 step reaction, it is at a disadvantage in terms of cost of facilities and operation. Furthermore, J. Wristers proposes a method of producing methylcyclopentane and cyclohexane from benzene using a HF-TaF5 catalyst at 50 degrees C, 14.6 kgf/cm², under presence of hydrogen, but the conversion ratio and formation ratio is unknown (J. Am. Chem. Soc. 97, 4312 (1975)).

Separate from the above, a method of reducing benzene contained in gasoline base material is proposed by hydrogenation and isomerization reactions. In Japanese Patent Laid-Open Number 7-216366, hydrogenation takes place with a nickel-on-alumina catalyst. The effluent is isomerized by a platinum-on-alumina catalyst. But, in this method, even though benzene is efficiently reduced, the rate of increase in naphthene hydrocarbons, especially methylcyclopentane, is low. As a result, there is still a problem of lightening. Furthermore, in Japanese Patent Laid-Open number 7-278569, a method of using a platinum supported MFI type aluminosilicate is disclosed. In this example, the reaction is finished in one step and the production rate of methylcyclopentane is high, but because the catalyst activity is high, the rate of decomposition reaction is also large, and this resulted in the problem of an increase of components with a carbon number less than or equal to 4.

US-A-2,721,226 discloses a method for converting aromatic hydrocarbons into alkyl cycloparaffins in the presence of hydrogen and an agent consisting essentially of silica-alumina carrying a hydrogenation catalyst. The catalyst is preferably nickel, although other metals such as cobalt, iron, vanadium, tungsten, chromium, platinum and palladium are mentioned.

US-A-3,974,061 discloses dual stage, vapour phase hydrosomerisation of hydrocarbon fractions containing C₅ and C₆ hydrocarbons using a chlorinated platinum catalyst on an alumina carrier.

### Disclosure of the invention

The object of the present invention is to solve the problems of the prior art. A further object is to provide a method for reducing benzene in the gasoline base material which does not need to separate benzene and which can not only hydrogenate and isomerize the raw material as is, but which also has few decomposition products with a carbon number less than or equal to 4. A further object is to efficiently obtain methylcyclopentane from crude benzene or benzene which is separated from the raw material. As a result, two main effects is obtained: 1) benzene reduction and its effective use 2) an increased concentration of methylcyclopentane which increases the acceleration responsiveness of the engine.

As a result of intensive study by the present inventors, there are conditions under which, when benzene is hydrogenated and isomerized in the presence of a solid super acid catalyst, benzene can be converted quantitatively to cyclohexane and methylcyclopentane. Furthermore, it has been found that the yield of methylcyclopentane is high. Furthermore, if the light fraction obtained from the catalytic reforming device is hydrogenated and isomerized in the presence of a solid super acid catalyst, there are hardly any decomposition products with a carbon number less than or equal to 4. It has been found that benzene is efficiently converted to methylcyclopentane, and the benzene content is reduced to 0.01% or less. The present invention is completed.

Isomerization and hydrogenation can use the same catalyst, or two or more types of catalysts with differing compositions filled into 2 or more layers can be used. By using a solid super acid catalyst, there is an advantage in that the reaction proceeds at low temperatures, and small amounts of catalyst will suffice. Furthermore, even when reaction temperatures are high, it has a characteristic where the amount of decomposition products of carbon number less than 4 is extremely small.

### Best Mode for Carrying Out the Invention

In the gasoline production of the present invention, examples of the starting substance which is the fraction containing benzene include: catalytic reformed light naphtha, light naphtha, cracked light gas, and the like. Among these, catalytic reformed naphtha is useable as a gasoline base material. Among catalytic reformed naphtha, the fraction containing large amounts of components with carbon numbers of 5∼7 is preferable. Such a fraction contains around 20∼40% benzene.

Of course, processing of benzene separated from catalytic reformed naphtha is also possible. When processing using the present catalyst, even with a catalytic reformed naphtha with low benzene content, with the isomerization of the straight chain paraffin to a branched paraffin, a secondary effect of an increased octane number is obtained.

Furthermore, light petroleum naphtha can also be used, although it has a low benzene content. The benzene contained in the light naphtha is around several percent. But, because the light naptha contains a large amount of straight chain paraffin, besides the action of converting benzene to methylcyclopentane, the straight chain paraffin can be isomerized to branched paraffin, as in the above, and the octane number can be greatly heightened.

Other benzene containing raw materials include: for example, cracked light oil, which is a by-product of ethylene production, and a carbonized oil produced when coal is dry distilled (carbonised). If crude benzene which contains 50-80% benzene or excess benzene which is obtained from dealkylation of aromatic hydrocarbons in the petrochemical industry can be obtained inexpensively, then these are converted to methylcyclopentane by hydrogenation and isomerization and can be used as a gasoline base.

The solid super acid catalyst used in the present inventions a catalyst comprising; 0.001 to 40 weight parts, in terms of a metal, of at least one metal selected from the group consisting of ruthenium, thodium, palladium, osmium, iridium and platinum, and/or at least one metal compound thereof; characterised in that the said catalyst further comprises 100 weight parts, in terms of oxide, of at least one hydrated oxide and/or oxide of at least one metal selected from the group consisting of titanium, zirconium and hafnium; 0.05 to 10 weight parts, in terms of sulphur, of a sulphureous component. The solid super acid catalyst indicates an acid with an acid strength greater than 100% sulfuric acid (Hₒ(Hammett's acid number = -11.93)).

The basic component is: at least one hydrated oxide and/or at least one oxide (hence abbreviated as the catalyst support) of at least one metal selected from the above mentioned group 4 metals, or else it is the catalyst support containing sulphurous component therein. There are a variety of methods for incorporating a sulphurous component into the catalyst support. A few examples are: impregnation with sulphurous component (Japanese patent publication numbers 59-6181 and 59-40056); mixing with ammonium sulphate in a solid phase (J.Chem.Soc.Comm.p.789, 1995).

The amount df sulphurous component which is impregnated should be, for every 100 weight parts of the catalyst support, 0.05-10 weight parts, preferably 1-10 weight parts, and more preferably 1-8 weight parts as sulphur. If it is less than 0.05 weight parts, the activity is not adequate. On the other hand, if it exceeds 10 weight parts, not only does the catalyst activity not increase further, the sulphuric acid which is released from the catalyst is increased, and therefore, this is not ideal.

In the catalyst support or the sulfureous component on-catalyst support, at least one metal selected from a group consisting of the above mentioned group 8 and 9 metals, and/or at least one metal compound thereof is incorporated. A known method of incorporation can be used for any of the metals. (For example, in Advance in Catalysis vol. 37, page 165, 1990, or the like). As a method for incorporating a group 6 metal, for example when incorporating tungsten or molybdenum, the method of Arata et al can be used (Japanese Patent Laid-Open number 1-288339, Proc.Int.Cong.Catal.9^{th}, vol.4, 1727 page, 1995). As a method for incorporating a group 6-10 metal and group 16 metal, for example when incorporating iron or manganese, methods were reported by Hino, Arata et al (Book of Abst.: 1995 Int.Chem.Congre.of Pacific Basin Soc.; Inorg.Chem.#160). When ruthenium, rhodium, palladium, osmium, iridium, or platinum is to be incorporated, the method in the report by Hino, Arata, et al can be used (Catalysis Letters Vo. 30 page 25, 1995). With these known methods, they can be used along, or can be used in combination.

There are no particular restrictions on the form of the above metal components when applying the metal components onto the catalyst support, but the metal components are preferably used in a form in which inclusion of catalytically poisonous components such as alkali metals is low. For example, these may be mentioned: oxides, chlorides, sulphates, mitrates, sulphides, ammonium salts of oxyacid, a complex with chloride ion, a complex with ammonium ion, a complex with cyanide ion, a complex with a thiocyanic acid, a compound complex with the above ions, an ammonium salt of the above complexes. After applying any of these onto the catalyst support, because the conditions change with baking and activation of the catalyst, the final form of the metal compound can be anything.

The amount of incorporation of group 6 and group 16 metals is 1∼40 weight parts, preferably 2∼35 weight parts, even more preferably 5∼30 weight parts, as metal versus 100 weight parts of the catalyst support. When it becomes lower than 1 weight part, the activity is not adequate. On the other hand, if it exceeds 40 weight parts, a decrease in activity is seen and is not ideal.

The amount of group 7 metal should be between 0.1∼15 weight parts, preferably 0.2∼12 weight parts, even more preferably 0.3∼10 weight parts in terms of metal relative to 100 weight parts of the catalyst support. If it becomes less than 0.1 weight parts, the activity is inadequate. On the other hand, if it exceeds 15 weight parts, a decrease in activity is seen and is not ideal.

The amount of group 8∼10 metals, for example iron, cobalt, and nickel, should be between 0.1∼15 weight parts, preferably 0.2∼12 weight parts, even more preferably 0.3∼10 weight parts, in terms of metal relative to 100 weight parts of the catalyst support. If It becomes less than 0.1 weight parts, the activity is inadequate. On the other hand, if it exceeds 15 weight parts, a decrease in activity is seen and is not ideal. With regard to ruthenium, rhodium, palladium, osmium, iridium, and platinum, the amount as metal should be 0.001∼10 weight parts, preferably 0.01∼7 weight parts, even more preferably 0.1∼5 weight parts, relative to 100 weight parts of the catalyst support. If it becomes less than 0.001 weight parts, the activity is inadequate. On the other hand, if it exceeds 10 weight parts, because the decomposition reaction becomes more prominent, it is not ideal.

Among these, solid super acid catalysts such as platinum supported sulfated zirconia, platinum supported sulfated iron oxide zirconia, and/or platinum and tungsten supported zirconia is most preferably used.

The above solid super acid catalysts may be used alone or in combination of two or more with differing compositions selected therefrom. Needless to say, each of these catalysts or mixtures thereof can be separately charged on the catalyst support to form two or more layers for use.

Because there are a plurality of contributing parameters, the hydrogenation and isomerization reaction conditions of the present invention can not be fixed to specific requirements. The conditions should be altered appropriately depending on the amount of raw material, or the like. But, with regard to the hydrogenation reaction, it is a volume reducing reaction where cyclohexane is generated from benzene and 3 molecules of hydrogen. It is also an exothermic reaction. As a result, in order to increase the amount of cyclohexane production, reaction pressure is preferably made high, and reaction temperature should be low. With respect to the isomerization reaction equilibrium between cyclohexane and methylcyclopentane, the higher the temperature, the greater the production of methylcyclopentane. From the above, if methylcyclopentane is attempted to be efficiently produced, an optimal temperature range exists. The reaction temperature should be 50∼400 degrees C, preferably 100∼350 degrees C, and more preferably 150∼330 degrees C. At temperatures lower than 50 degrees C, the reaction rate is slow, and the production amount can not be guaranteed. With temperatures greater than 400 degrees C, the decomposition reaction becomes more prominent. The production of gaseous hydrocarbons with carbon numbers of 3∼4 increases. Therefore, when gaseous hydrocarbons are unnecessary, it is preferable to have temperatures not higher than 400 degrees C.

The reaction pressure is the higher the better, but when considering operation efficiency, the pressure should be 4.0 x 10 ⁵ ∼1.5 x 10⁷ Pa (gage pressure approximately 3∼150 kgf/cm²). Preferably it should be 5.9 x 10⁵ ∼ 9.8 x 10⁶ Pa (gage pressure approximately 5 ∼ 100 kgf/cm²). If the pressure goes below 4.0 x 10 ⁵ Pa, the reaction rate is low. When pressure exceeds 1.5 x 10⁷ Pa, it is no longer preferable in terms of operational safety.

An appropriate value for liquid space velocity can be chosen, taking into consideration temperature and pressure. Normally it is 0.2 - 10 h^{-1,}ideally it is 0.5 ∼ 6 h⁻¹. Similarly, with regard to hydrogen/raw material volume ratio, an appropriate value can be selected, but normally it is 100 ∼ 10,000. Preferably it is 300 ∼ 5000. With regard to the reaction device, isomerization and hydogenation can be conducted in a single step with a single reaction device, or isomerization and hydrogenation can be conducted in two steps with two reaction devices.

### Embodiments

### Embodiment 1

100g of zirconium hydroxychloride was dissolved in 2000ml of distilled water. Precipitate was generated by adding 28% aqueous ammonia until a pH of 8 was reached. After filtering, washing with water, and drying the precipitate, a white powder of dried hydrated zirconia was obtained. After contacting 300 ml of 0.5 mol/l sulfuric acid solution to 20 g of dried hydrated zirconia, excess sulfuric acid was removed by filtration, and it was dried at 100 degrees C. Next, after impregnating the sulfated zirconia with 3.0 ml of solution containing 0.38 g of chloroplatinic acid 6 hydrate, it was dried and baked at 590 degrees C. A platinum supported sulfated zirconia was obtained. The composition for this catalyst is the following: for 100 weight parts calculated as zirconium oxide of a zirconia component, 2.1 weight parts calculated as sulfur of sulfureous component, and 0.7 weight parts calculated as platinum of a platinum compound. After forming this catalyst into a pellet shape, it was pulverized, and the resultant powder was sifted with a 10∼20 mesh and charged in an amount of 4 ml onto a catalyst layer. It then underwent a reduction process for 1.5 hours at a pressure of 5.9 x 10⁵ Pa (about 5 kgf/cm² gage pressure) and temperature of 300 degrees C and a hydrogen flow rate of 50 ml/min. Afterwards, hydrogenation and isomerization were conducted under the conditions of 3.0 x 10⁶ Pa (gage pressure of approximately 30 kgf/cm²), liquid space velocity of 2.0 h⁻¹, temperature of 230 degrees C, hydrogen/ stock oil 500 l/l. The stock oil used therein was the fraction after catalytic reforming which has as its main component a 6 carbon (C6) hydrocarbon. The experimental conditions are shown in Table 1. The stock oil composition and the yield of products are shown in Table 2. The analysis of each component was conducted using a gas chromatograph with a FID detection device. In some cases, gas chromatography could not be used to analyze benzene and toluene at a 0.01% level. In such a case, analysis using high speed liquid chromatography was conducted.

**Table 1**

| | Catalyst | Stock oil | Reaction temp. (°C) | Liquid space velocity (h⁻¹) | H₂/stock oil (l/l) | Reaction pressure (Pa) |
|---|---|---|---|---|---|---|
| Embodiment 1 | Platinum-supported sulfated zirconia | Fraction mainly C6 after catalytic reforming | 230 | 2.0 | 500 | 3.0x10⁶ |
| Embodiment 2 | " | " | 200 | 1.3 | 850 | 5.9x10⁵ |
| Embodiment 3 | " | " | 200 | 1.5 | 1700 | 5.9x10⁵ |
| Embodiment 4 | " | " | 300 | 1.7 | 850 | 5.9x10⁵ |
| Embodiment 5 | " | " | 200 | 2.7 | 850 | 5.9x10⁵ |
| Embodiment 6 | " | " | 200 | 2.0 | 500 | 1.1x10⁶ |
| Embodiment 7 | " | " | 200 | 2.0 | 500 | 2.1x10⁶ |
| Embodiment 8 | " | " | 200 | 2.0 | 500 | 3.0x10⁶ |
| Embodiment 9 | " | Benzene | 240 | 1.1 | 2000 | 5.9x10⁵ |
| Embodiment 10 | " | " | 310 | 1.1 | 2000 | 5.9x10⁵ |
| Embodiment 11 | " | Crude benzene | 230 | 1.5 | 750 | 3.0x10⁶ |
| Embodiment 12 | " | " | 230 | 1.5 | 750 | 3.0x10⁶ |

The abbreviations used in Table 2 and beyond are listed below.
C3: fraction with carbon number 3
C4: fraction with carbon number 4
branched C5: fraction with isoparaffin with a carbon number 5
n-C5: faction with n-paraffin with a carbon number 5
MCP: methylcyclopentane
CH: cyclohexane
branched C6: fraction with isoparaffin with a carbon number 6
n-C6: fraction with n-paraffin with a carbon number 6
MCH: methylcyclohexane
branched C7: fraction with isoparaffin with a carbon number 7
H²/oil: ratio of feed hydrogen to feed stock oil

**Table 2**

| | Concentration (weight %) | | | | | |
|---|---|---|---|---|---|---|
| | Stock oil | Embodiment 1 | Embodiment 2 | Embodiment 3 | Embodiment 4 | Embodiment 5 |
| C3 | 0.0 | 0.2 | 1.9 | 1.3 | 3.2 | 0.9 |
| C4 | 0.0 | 1.3 | 2.6 | 2.1 | 4.9 | 1.6 |
| Branched C5 | 0.4 | 1.0 | 0.5 | 0.5 | 1.1 | 0.6 |
| n - C5 | 0.3 | 0.3 | 0.3 | 0.3 | 0.4 | 0.3 |
| MCP | 1.3 | 25.6 | 18.5 | 17.0 | 12.7 | 14.4 |
| CH | 1.2 | 9.0 | 12.6 | 13.5 | 15.9 | 16.6 |
| Branched C6 | 20.6 | 32.8 | 25.9 | 24.0 | 16.7 | 25.5 |
| n - C6 | 11.8 | 7.6 | 12.7 | 11.4 | 9.9 | 12.7 |
| MCH | 18.8 | 8.9 | 12.6 | 13.8 | 10.7 | 14.4 |
| Branched C7 | 7.6 | 10.5 | 9.7 | 10.9 | 7.6 | 9.8 |
| n - C7 | 4.7 | 2.8 | 3.7 | 6.2 | 2.8 | 3.7 |
| Benzene | 30.7 | ≤0.01 | ≤0.01 | ≤0.01 | 13.4 | ≤0.01 |
| Toluene | 2.6 | ≤0.01 | ≤0.01 | ≤0.01 | 0.7 | ≤0.01 |

### Embodiments 2-3

The embodiments were tested in the same manner as embodiment 1, except that the liquid space velocity was 1.3 and 1.5 h⁻¹, reaction temperature was at 200 degrees C, hydrogen to stock oil ratio was 850 and 1700 l/l, pressure was 5.9 x 10⁵ Pa (approximately 5 kg f / cm² by gage pressure). Experimental conditions are shown in Table 1, and results are shown in Table 2.

### Embodiment 4

The embodiment was tested in the same manner as embodiment 1, except that the liquid space velocity was 1.7 h⁻¹, reaction temperature was at 300 degrees C, hydrogen to stock oil ratio was 850 l/l, pressure was 5.9 x 10⁵ Pa (approximately 5 kg f/ cm² by gage pressure). Experimental conditions are shown in Table 1, and results are shown in Table 2. Due to the high reaction temperature, it can be seen that the decomposition reaction was somewhat increased.

### Embodiment 5

The embodiment was tested in the same manner as embodiment 1, except that the liquid space velocity was 2.7 h⁻¹, reaction temperature was 200 degrees C, hydrogen to stock oil ratio was 850 l/l, pressure was 5.9 x 10⁵ Pa (approximately 5 kg f/ cm² by gage pressure). Experimental conditions are shown in Table 1, and results are shown in Table 2.

### Embodiments 6-8

The embodiments were tested in the same manner as embodiment 1, except that the liquid space velocity was 2.0 h⁻¹, reaction temperature was 200 degrees C, hydrogen to stock oil ratio was 850 l/l, pressure was 1.1 x 10⁶ Pa, 2.1 x 10⁶ Pa, 3.0 x 10⁶ Pa (approximately 10, 20, and 30 kg f/ cm² by gage pressure, respectively). Experimental conditions are shown in Table 1, and results are shown in Table 3. It is clear that as pressure increases, the production amount of methylcyclopentane increases.

**Table 3**

| | Concentration (mass %) | | |
|---|---|---|---|
| | Embodiment 6 | Embodiment 7 | Embodiment 8 |
| C3 | 0.2 | 0.3 | ≤0.05 |
| C4 | 0.7 | 1.7 | 2.0 |
| Branched C5 | 0.6 | 0.9 | 0.6 |
| n - C5 | 0.3 | 0.2 | 0.2 |
| MCP | 15.8 | 19.2 | 24.6 |
| CH | 13.0 | 13.0 | 11.5 |
| Branched C6 | 19.9 | 19.4 | 20.4 |
| n - C6 | 11.3 | 10.6 | 9.9 |
| MCH | 17.5 | 17.2 | 17.5 |
| Branched C7 | 10.9 | 10.6 | 9.5 |
| n - C7 | 4.5 | 4.0 | 3.8 |
| Benzene | 5.2 | 2.9 | ≤0.01 |
| Toluene | 0.1 | ≤0.01 | ≤0.01 |

### Embodiments 9-10

Using benzene as a raw material (guaranteed reagent with 99.5% purity), the embodiments were tested in the same manner as embodiment 1, except that the liquid space velocity was 1.1 h⁻¹, hydrogen to stock oil ratio was 2000 l/l, pressure was 5.9 x 10⁵ Pa (approximately 5 kg f/ cm² by gage pressure), reaction temperature was 240 and 310. Experimental conditions are shown in Table 1, and results are shown in Table 4. It can be seen from the results that when reaction temperature is high, the hydrogenation and isomerization reactions of benzene do not proceed easily.

**Table 4**

| | Concentration (mass %) | | |
|---|---|---|---|
| | Stock oil | Embodiment 9 | Embodiment 10 |
| C3 | - | ≤0.05 | ≤0.05 |
| C4 | - | 0.1 | 0.1 |
| Branched and n-C5 | - | 0.3 | 0.1 |
| MCP | - | 60.0 | 46.3 |
| CH | - | 39.6 | 11.0 |
| Branched C6 | - | ≤0.05 | 2.0 |
| n - C6 | - | ≤0.05 | 0.7 |
| MCH | - | ≤0.05 | ≤0.05 |
| Branched C7 | - | ≤0.05 | ≤0.05 |
| n - C7 | - | ≤0.05 | ≤0.05 |
| Benzene | 100 | ≤0.01 | 39.8 |
| Toluene | - | ≤0.01 | ≤0.01 |

### Embodiments 11∼12

Stock oils which simulated crude benzene (70% benzene + 30% toluene and 60% benzene + 40% toluene. In these stock oils, benzene was a guaranteed reagent with greater than 99.5% purity, toluene was a guaranteed reagent with greater than 98% purity) were used. Each embodiment was tested in the same manner as embodiment 1, except that the liquid space velocity was 1.5 h⁻¹, hydrogen to stock oil ratio was 750 l/l, pressure was 3.0 x 10⁶ Pa (approximately 30 kg f cm² by gage pressure), reaction temperature was 230. Experimental conditions are shown in Table 1, and results are shown in Table 5.

**Table 5**

| | Concentration (mass %) | | | |
|---|---|---|---|---|
| | Embodiment 11 | | Embodiment 12 | |
| | Stock oil | Product yield | Stock oil | Product yield |
| C3 | - | ≤0.05 | - | ≤0.05 |
| C4 | - | 0.1 | - | 0.1 |
| Branched and n-C5 | - | 0.2 | - | 0.1 |
| MCP | - | 38.0 | - | 32.5 |
| CH | - | 26.3 | - | 23.3 |
| Branched and n-C6 | - | 0.3 | - | 0.2 |
| MCH | - | 18.2 | - | 21.5 |
| Branched C7 | - | 10.0 | - | 13.2 |
| n - C7 | - | 2.1 | - | 3.5 |
| Benzene | 70 | ≤0.01 | 60 | ≤0.01 |
| Toluene | 30 | 4.8 | 40 | 5.6 |

### Industrial Applicability

By the present invention, benzene can be efficiently converted to methylcyclopentane in benzene or a fraction which contains benzene. Using the method in the present invention, light naphtha can be converted to an isomerized product with a higher octane number. The benzene which is hazardous is converted to methylcyclopentane. As a result, the reduced benzene is effectively used, and a gasoline with a high octane number and which has good acceleration can be produced.

## Claims

1. A production method for a hydrocarbon containing methylcyclopentane, wherein: benzene or a benzene containing hydrocarbon is hydrogenated and isomerized in the presence of a solid super acid catalyst, said solid super acid catalyst comprises :
(b) 0.001 to 40 weight parts, in terms of a metal, of at least one metal selected from the group consisting of ruthenium, rhodium, palladium, osmium, iridium and platinum, and/or at least one metal compound thereof; and **characterised in that** the said catalyst further comprises
(a) 100 weight parts, in terms of oxide, of at least one hydrated oxide and/or oxide of at least one metal selected from the group consisting of titanium, zirconium and hafnium;
(c) 0.05 to 10 weight parts, in terms of sulphur, of a sulphurous component.

2. A production method for a hydrocarbon containing methylcyclopentane as claimed in Claim 1, wherein: the benzene content is reduced to 0.01% or less by the production method.

3. A production method for a hydrocarbon containing methylcyclopentane as claimed in Claim 1, or Claim 2, wherein: said at least one metal selected from the group consisting of titanium, zirconium and hafnium is zirconium.

4. A production method for a hydrocarbon containing methylcyclopentane as claimed in any of Claims 1 to 3, wherein: said at least one metal is selected from the group consisting of ruthenium, rhodium, palladium, osmium, iridium and platinum.

5. A production method for a hydrocarbon containing methylcyclopentane as claimed in any preceding claim, wherein: sulphuric acid or ammonium sulphate is incorporated as a source of said sulphureous component into the hydrated oxide and/or oxide of at least one metal selected from the group consisting titanium, zirconium and hafnium.

6. A production method for a hydrocarbon containing methylcyclopentane as claimed in any preceding claim, wherein: said solid super acid catalyst is prepared by calcining said component (a) incorporating sulphuric acid or ammonium sulphate as said component (c) therein and supporting said component (b) thereon.

## Patentansprüche

1. Verfahren zur Herstellung eines Methylcyclopentan enthaltenden Kohlenwasserstoffs,
**worin** Benzol oder ein Benzol enthaltender Kohlenwasserstoff in Gegenwart eines festen Supersäurekatalysators hydriert und isomerisiert wird, wobei der feste Supersäurekatalysator umfasst:
(b) 0,001 bis 40 Gew.-Teile, bezogen auf das Metall, von wenigstens einem aus der Gruppe bestehend aus Ruthenium, Rhodium, Palladium, Osmium, Iridium und Platin ausgewählten Metall und/oder von wenigstens einer Metallverbindung davon; wobei der Katalysator des Weiteren umfasst;
(a) 100 Gew.-Teile, bezogen auf das Oxid, von wenigstens einem hydrierten Oxid und/oder einem Oxid aus wenigstens einem aus der Gruppe bestehend aus Titan, Zirkonium und Hafnium ausgewählten Metall;
(c) 0,05 bis 10 Gew.-Teile, bezogen auf den Schwefel, einer schwefelhaltigen Komponente.

2. Veffahren zur Herstellung eines Methylcyclopentan enthaltenden Kohlenwasserstoffs nach Anspruch 1,
**worin** der Benzolgehalt durch das Herstellungsverfahren auf 0,01 % oder weniger verringert wird.

3. Verfahren zur Herstellung eines Methylcyclopentan enthaltenden Kohlenwasserstoffs nach Anspruch 1 oder 2,
**worin** das wenigstens eine aus der Gruppe bestehend aus Titan, Zirkonium und Hafnium ausgewählte Metall Zirkonium ist.

4. Verfahren zur Herstellung eines Methylcyclopentan enthaltenden Kohlenwasserstoffs nach einem der Ansprüche 1 bis 3,
**worin** das wenigstens eine Metall aus der Gruppe bestehend aus Ruthenium, Rhodium, Palladium, Osmium, Iridium und Platin ausgewählt ist.

5. Verfahren zur Herstellung eines Methylcyclopentan enthaltenden Kohlenwasserstoffs nach einem der vorhergehenden Ansprüche,
**worin** Schwefelsäure oder Ammoniumsulfat als Quelle der schwefelhaltigen Komponente in das hydrierte Oxid und/oder das Oxid aus wenigstens einem aus der Gruppe bestehend aus Titan, Zirkonium und Hafnium ausgewählten Metall eingearbeitet wird.

6. Verfahren zur Herstellung eines Methylcyclopenthan enthaltenden Kohlenwasserstoffs nach einem der vorhergehenden Ansprüche,
**worin** der feste Supersäurekatalysator durch Calcinieren der Komponente (a), Einarbeiten von Schwefelsäure oder Ammoniumsulfat als Komponente (c) darin und Auftragen der Komponente (b) darauf hergestellt wird.

## Revendications

1. Procédé de production pour un hydrocarbure contenant du méthylcyclopentane, dans lequel : le benzène ou un hydrocarbure contenant du benzène est hydrogéné et isomérisé en présence d'un catalyseur superacide solide, ledit catalyseur superacide solide comprend :
(b) 0,001 à 40 parties en poids, exprimées en métal, d'au moins un métal choisi parmi le groupe constitué du ruthénium, du rhodium, du palladium, de l'osmium, de l'iridium et du platine, et/ou au moins d'un composé métallique de celui-ci, et **caractérisé en ce que** ledit catalyseur comprend en outre
(a) 100 parties en poids, exprimées en oxyde, d'au moins un oxyde hydraté et/ou d'un oxyde d'au moins un métal choisi parmi le groupe constitué du titane, du zirconium et du hafnium,
(c) 0,05 à 10 parties en poids, exprimées en soufre, d'un composé sulfureux.

2. Procédé de production pour un hydrocarbure contenant du méthylcyclopentane selon la revendication 1, dans lequel : la teneur en benzène est réduite à 0,01 % ou moins par le procédé de production.

3. Procédé de production pour un hydrocarbure contenant du méthylcyclopentane selon la revendication 1 ou la revendication 2, dans lequel : ledit au moins un métal choisi parmi le groupe constitué du titane, du zirconium et du hafnium est le zirconium.

4. Procédé de production pour un hydrocarbure contenant du méthylcyclopentane selon l'une quelconque des revendications 1 à 3, dans lequel : ledit au moins un métal est choisi parmi le groupe constitué du ruthénium, du rhodium, du palladium, de l'osmium, de l'iridium et du platine.

5. Procédé de production pour un hydrocarbure contenant du méthylcyclopentane selon l'une quelconque des revendications précédentes, dans lequel : de l'acide sulfurique ou du sulfate d'ammonium est incorporé en tant que source dudit composant sulfureux dans l'oxyde hydraté et/ou l'oxyde d'au moins un métal choisi parmi le groupe constitué du titane, du zirconium et du halfnium.

6. Procédé de production pour un hydrocarbure contenant du méthylcyclopentane selon l'une quelconque des revendications précédentes, dans lequel : ledit catalyseur superacide solide est préparé en calcinant ledit composant (a), en incorporant de l'acide sulfurique ou du sulfate d'ammonium en tant que dit composant (c) dans celui-ci et en supportant ledit composant (b) sur celui-ci.
